# EUROPÄISCHE PATENTSCHRIFT

(11) **EP 3 206 386 B1**
(45) Veröffentlichungstag und Bekanntmachung des Hinweises auf die Patenterteilung: **21.11.2018**
(21) Anmeldenummer: 17163342.3
(22) Anmeldetag: 14.02.2014
(51) Int. Cl.: H04N 5/225, A61B 1/04, A61B 1/00, A61B 1/005, A61B 1/05, G02B 23/24, H01L 27/146

(54) **BAUGRUPPE FÜR EIN VIDEOENDOSKOP**
ASSEMBLY FOR A VIDEO ENDOSCOPE
MODULE POUR UN VIDÉO-ENDOSCOPE

(30) Priorität: 15.02.2013 DE 102013202516
(43) Veröffentlichungstag der Anmeldung: 16.08.2017
(62) Teilanmeldung aus: 14704359.0
(73) Patentinhaber: Richard Wolf GmbH, 75438 Knittlingen (DE)
(72) Erfinder: Handte, René, 71665 Vaihingen/enz (DE); Heimberger, Rudolf, 75038 Oberderdingen (DE)
(74) Vertreter: Patentanwälte Vollmann & Hemmer

(56) Entgegenhaltungen:
- EP-A1- 2 677 736
- WO-A1-2009/041724
- JP-A- S56 144 673
- JP-A- 2011 217 887
- US-A1- 2004 167 378
- US-A1- 2007 171 012
- US-A1- 2011 249 106
- US-A1- 2012 310 043
- US-B1- 6 635 865

## Beschreibung

Die Erfindung betrifft eine Baugruppe für ein Videoendoskop mit den im Oberbegriff des Anspruchs 1 angegebenen Merkmalen.

Bei Videoendoskopen zählt es zum Stand der Technik, eine Baugruppe bestehend aus Bildsensor, Kondensator, Platine und Kabel im distalen Bereich direkt hinter dem Objektiv zu platzieren. Das durch das Objektiv erzeugte Bild wird auf den Sensor projiziert und die Signale werden über durch den Endoskopschaft hindurch geführte Kabel an die proximalseitig angeordnete Elektronik weitergeleitet. Derartige Endoskope bzw. Technoskope sind auch unter dem Begriff "Chip on the Tip-Endoskope" bekannt. Dabei gibt es unterschiedliche Bauarten, so kann der Bildsensor stehend oder liegend angeordnet sein. Auch bei Endoskopen dieser Bauart ist man stets bemüht, die Querschnittsabmessungen klein zu halten, um durch möglichst kleine Öffnungen hindurchgelangen zu können. Je kleiner jedoch der Durchmesser des Endoskopes ist, desto länger wird die Baulänge im distalen Endbereich, was insbesondere bei flexiblen Endoskopen von Nachteil ist, da die Endoskope in dem Endbereich, in dem diese Baugruppe angeordnet ist, starr, d. h. unflexibel sind. Diese Problematik stellt sich bei starren Endoskopen nicht, allerdings ist man stets bemüht, unabhängig von der Bauform nach Möglichkeit baugleiche Baugruppen verwenden zu können.

Aus WO 2009/041724 A1 zählt ein derartiges Videoendoskop zum Stand der Technik. Dort ist der Bildsensor quer zur Längsachse des Endoskops und auf einer flexiblen mehrfach abgebogenen Platine angeordnet.

Eine ähnliche Anordnung ist aus US 2011/0249106 A1 bekannt. Auch dort ist der Bildsensor stehend, also quer zur Längsachse des Endoskops auf einer flexiblen Platine angeordnet, welche weitere elektronische Bauteile trägt und um ein Stützelement herum angeordnet ist.

Vor diesem Hintergrund liegt der Erfindung die Aufgabe zugrunde, eine gattungsgemäße Baugruppe für ein Videoendoskop so auszugestalten, dass einerseits eine möglichst kleine Baugröße im Bezug auf die vorgegebene Sensorgröße, also eine möglichst kleine Querschnittsab-messung und andererseits eine kurze Baulänge erreicht wird. Darüber hinaus soll eine kostengünstige Herstellung und eine Verwendung sowohl in starren als auch in flexiblen Endoskopen möglich sein.

Diese Aufgabe wird gemäß der Erfindung durch eine Baugruppe für ein Videoendoskop mit den in Anspruch 1 angegebenen Merkmalen erreicht. Vorteilhafte Ausgestaltungen der Erfindung sind in den Unteransprüchen, der nachfolgenden Beschreibung und der Zeichnung angegeben. Dabei können gemäß der Erfindung die in den Unteransprüchen und der Beschreibung angegebenen Merkmale jeweils für sich, aber auch in geeigneter Kombination, die erfindungsgemäße Lösung gemäß Anspruch 1 weiter ausgestalten.

Die erfindungsgemäße Baugruppe für ein Videoendoskop weist einen bildaufnehmenden Sensor auf, der elektrisch leitend auf einer flexiblen Platine angeordnet ist, welche neben dem Sensor abgebogen und proximalseitig des Sensors mit mindestens einem elektrischen Kabel leitend verbunden ist. Der Grundgedanke der vorliegenden Erfindung ist es somit, durch eine flexible Platine, wie sie typischerweise in sogenannter "Dünnschichttechnik" hergestellt wird, sowohl den Sensor als auch den oder die Kabel anzubringen, wobei die Platine die leitenden Verbindungen zwischen Sensor und Kabelanschlüssen bildet und die Kontaktierung der Platine vorteilhaft von nur einer Seite erfolgt, sodass sowohl Sensor als auch Kabelanschlüsse auf derselben Seite kontaktiert werden können, jedoch durch Abbiegen der Platine räumlich auf unterschiedlichen Ebenen befindlich sind, typischerweise die Kabelanschlüsse proximalseitig des Sensors. Hierdurch ist es möglich, eine sehr kompakt und klein bauende Baugruppe zu schaffen, die darüber hinaus kostengünstig herstellbar ist. Dabei werden die für den Sensor und die Kabel erforderlichen Kontaktflächen sowie ggf. weitere Kontaktflächen für elektronische Bauteile durch einen schichtweisen Aufbau hergestellt. Die elektrische Verschaltung ist in diesen schichtweisen Aufbau integriert, sodass die Platine mit allen notwendigen Leitern, Durchkontaktierungen, der Schaltung, der Isolation und den Lötpads eine Dicke zwischen 40 und 100 µm aufweisen kann. Dabei liegen sämtliche Kontakte nur auf einer Seite, die Kontaktierung proximalseitig des Sensors ist somit durch das Umbiegen erst möglich. Dabei kann der Aufbau der Platine auf einer planen Platte, beispielsweise einer Glasplatte schichtweise erfolgen, wobei die Bauteilbestückung vorteilhaft ebenfalls direkt auf dieser Platte erfolgt. Durch Biegen der Platine an vorteilhaft durch Markierungen gekennzeichneten Stellen kann diese sehr kompakt zusammengefaltet werden, wodurch man aus der mit dem Sensor und ggf. weiteren elektronischen Bauteilen bestückten und relativ langen Platine eine räumlich kompakte Baugruppe mit kleinstmöglichen Abmaßen erhält, und das sowohl in radialer als auch in axialer Richtung.

Gemäß der Erfindung weist die Platine neben dem Sensor mindestens ein weiteres elektronisches Bauteil auf, welches auf einem abgebogenen Teil der Platine proximalseitig des Sensors angeordnet ist. Ein solches Bauteil ist typischerweise ein Kondensator, der in diesem proximalen Baugruppenteil erforderlich ist und vergleichsweise großen Raumbedarf hat. Dieser wird daher vorzugsweise auf der Platine neben dem Sensor angeordnet und durch Abbiegen der Platine proximalseitig des Sensors verbracht.

Besonders vorteilhaft wird die erfindungsgemäße Baugruppe durch eine lang gestreckte schmale Platine gebildet werden, welche etwa die Breite des Sensors aufweist und zu zwei Seiten des Sensors nämlich zu zwei abgewandt gegenüberliegenden Seiten und mehrfach abgebogen ist, sodass sich ein schlanker Körper ergibt, dessen Stirnseite durch den Sensor gebildet ist.

Um die Platine insbesondere zerstörungsfrei um 180° biegen zu können, ist gemäß der Erfindung ein Kern vorgesehen, um den die Platine herum gebogen ist, welcher in dem Bereich fluchtend zum Sensor rückseitig der Platine angeordnet ist.

Darüber hinaus ist es zweckmäßig, die Platine im Bereich des Sensors verstärkt auszubilden. Hierzu ist gemäß der Erfindung die Platine rückseitig in dem Bereich, wo vorderseitig der Sensor angeordnet ist, durch einen Stützkörper verstärkt, der z. B. rückseitig der Platine aufgeklebt oder in anderer geeigneter Weise fixiert ist, dabei werden Stützkörper und Kern durch denselben Körper gebildet.

Dabei ist es besonders vorteilhaft, wenn die Platine nur auf einer Platinenseite den Sensor und weitere elektronische Bauteile und Anschlüsse trägt, da dann eine besonders dünne und vorteilhaft einlagige Platine Verwendung finden kann. Erfindungsgemäß weist die Baugruppe eine vorzugsweise einlagige Flex-Platine auf, die eine Dicke zwischen 40 µm und 100 µm, beispielsweise 60 µm aufweist. Solche extrem dünnen Platinen können in besonders engen Radien abgebogen werden, und zwar auch über 90° und mehr, sodass quasi eine Faltung der Platine möglich ist, wodurch das Bauvolumen reduziert wird. Gemäß der Erfindung kann eine solche extrem dünne und flexible Platine in einem Biegeradius abgebogen sein, der dem 0,5 bis 2fachen der Platinendicke entspricht. Vorteilhaft entspricht der Biegeradius sogar nur dem 0,8 bis 1,2fachen der Platinendicke, sodass beispielsweise bei einer Platinendicke von 40 µm auch ein extrem kleiner Biegeradius von 40 µm vorgesehen sein kann.

Um im Bereich einer Biegung die Biegespannung innerhalb der Platine zu reduzieren, ist gemäß einer vorteilhaften Weiterbildung der Erfindung mindestens eine Ausnehmung in diesem Bereich vorgesehen. Zweckmäßigerweise sind im Biegebereich mehrere nebeneinander angeordnete Ausnehmungen vorgesehen, die bevorzugt eine gerundete Kontur aufweisen und somit Kräfte kontinuierlich in die verbleibenden Stegbereiche einleiten können. Wenn im Biegebereich aufgrund der Leiterbahnen kein Raum für eine solche Ausnehmung ist, empfiehlt es sich, zumindest in den Randbereichen seitlich offene Ausnehmungen vorzusehen, sodass die Platine im Biegebereich eine geringere Breite als im übrigen Bereich aufweist.

Gemäß einer vorteilhaften Weiterbildung der Erfindung sind die Platinenteile, die rückseitig aneinander anliegen, vorteilhaft durch Kleben miteinander verbunden, sodass insbesondere vor dem Verguss der Baugruppe eine höhere Stabilität gegeben ist.

Eine besonders kompakte und für eine Anordnung mit stehendem Sensor vorgesehene Baugruppe ergibt sich dadurch, dass die Platine an einer Seite neben dem Sensor um etwa 180° abgebogen ist, dann sensorparallel verläuft und einen davon um etwa 90° proximalwärts abgebogenen Endabschnitt aufweist, der an dem sensorparallelen Abschnitt anschließt. Dann ergeben sich konstruktiv noch Flächen auf der Platine, die für weitere Bauteile oder Anschlüsse genutzt werden können. Ein solches weiteres Bauteil kann insbesondere ein Kondensator sein, der dann vorteilhaft im Endabschnitt auf der Platine elektrisch leitend angeordnet ist, also den Raum zwischen der Platine und der durch die 180°-Umbiegung gebildeten Seite, also den Freiraum rückseitig des Sensors jenseits der Platine nutzt.

Vorzugsweise erfolgt die elektrische Verbindung nicht über ein gemeinsames Kabel, sondern über mehrere einadrige Kabel, die an Lötpads, die an einem oder beiden Endabschnitten der Platine angeordnet sind, elektrisch leitend angeschlossen sind. Durch das Verwenden von Einzelleitern anstelle eines kompakten und fertig konfektionierten Kabelverbundes ist es möglich, die Verbindung der elektrischen Leiter mit der Platine sehr kurz zu gestalten. Dies wird dadurch möglich, dass die Einzelleiter sehr kurz abisoliert und mit der Platine verlötet werden können, wie dies bei einem kompakten mehradrigen Kabel, bei welchem die einzelnen Leiter fest eingebunden sind, so nicht möglich ist.

Um die Vielzahl dieser ein- oder wenigadrigen Kabel zusammenzufassen und zu bündeln, ist gemäß einer Weiterbildung der Erfindung vorgesehen, entweder nur die Kabel oder aber vorzugsweise die platinenseitigen Kabelenden und die Endabschnitte der Platine durch einen abschirmenden Schlauch zu umgeben.

Alternativ kann die Platine auch bis zum proximalseitigen Ende des Videoendoskops geführt sein und so die Kabel in diesem Bereich ersetzen, dann erfolgt die Kontaktierung im Bereich des proximalseitigen Schaftendes. Das Objektiv wird vorteilhaft unmittelbar auf dem Sensor angebracht. Diese ganze Baugruppe, bestehend aus ggf. Objektiv, Sensor, Platine und den platinenseitigen Kabelenden mit dem abschirmenden Schlauch, wird dann in einem umgebenden Gehäuse in Kunststoff vergossen, um die Bauteile zueinander zu fixieren und gleichzeitig vor äußeren Einflüssen zu schützen.

Die erfindungsgemäße Baugruppe ist zum Eingliedern am distalen Ende eines Endoskops mit einem starren oder flexiblen Schaft vorgesehen, wobei durch den Schaft die elektrischen Verbindungskabel geführt sind oder aber die Platine selbst die Leitungsverbindungen in diesem Bereich bildet.

Die Erfindung ist nachfolgend anhand eines in der Zeichnung dargestellten Ausführungsbeispiels näher erläutert. Es zeigen:
- Fig. 1: in stark vereinfachter schematischer Längsschnittdarstellung das distale Ende eines Endoskops,
- Fig. 2: eine in einem Gehäuse eingegliederte Baugruppe mit Objektiv in perspektivischer Ansicht von vorne,
- Fig. 3: in vergrößerter Darstellung die Baugruppe aus Fig. 1 in ein Gehäuse eingegossen mit daran angeschlossenen Kabeln von vorne,
- Fig. 4: die Baugruppe ohne Gehäuse in perspektivischer Ansicht von hinten,
- Fig. 5: eine Draufsicht auf die bestückte Platine von der Sensorseite,
- Fig. 6: eine Seitenansicht der bestückten Platine,
- Fig. 7: die bestückte Platine in abgebogener Form,
- Fig. 8: ein Blockschaltbild der Baugruppe und
- Fig. 9: einen Stromlaufplan der Baugruppe.

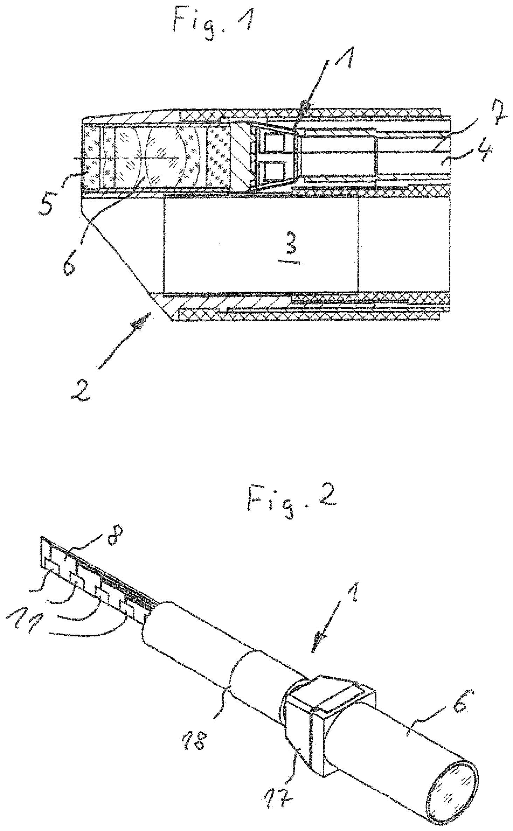

In Figur 1 ist beispielhaft das distale Ende eines Videoendoskops im Längsschnitt dargestellt, welches mit einer erfindungsgemäßen Baugruppe 1 ausgestattet ist. In der Figur zu sehen ist der distale Endabschnitt 2 mit einem Arbeitskanal 3 und einem Optikkanal 4, der endseitig durch ein fest und dicht den Kanal abschließendes Fenster 5, z. B. aus Saphir, abgeschlossen wird. An dieses Fenster 5 schließt sich proximalseitig ein Objektiv 6, welches fest mit dem distalen Ende der Baugruppe 1 verbunden ist, deren elektrische Anschlusskabel in Form von einzelnen isolierten und von einem abschirmenden Schlauch umgebenen Drähten 12 in Form eines in Figur 1 nur schematisch dargestellten Kabels 7 zum proximalen Instrumentenende geführt sind.

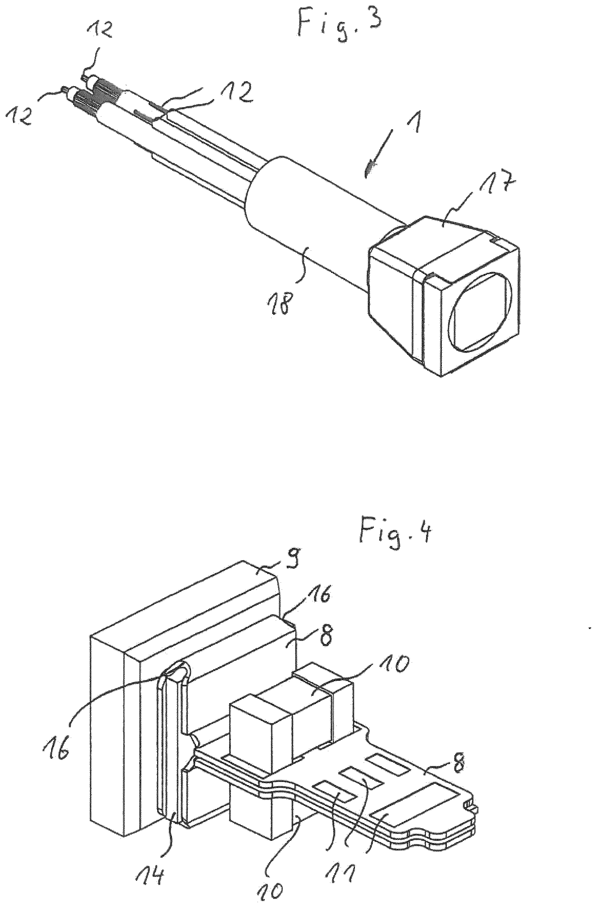

Die Baugruppe 1 ist anhand von Figur 2 (mit Objektiv) in abgewandter Form und Figur 3 (ohne Objektiv) dargestellt.

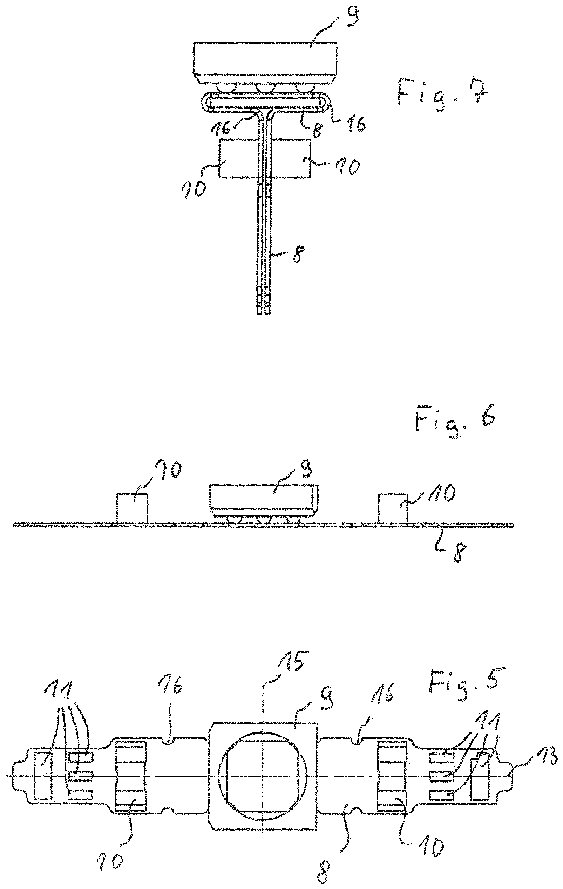

Aufgebaut ist eine solche Baugruppe 1 auf einer lang gestreckten, hochflexiblen Platine 8, wie sie in den Figuren 4 - 7 dargestellt ist, und zwar in den Figuren 5 und 6 in noch planer Form, in welcher die Bestückung erfolgt. Die Platine 8 hat eine im Wesentlichen lang gestreckte und rechteckige Form. Sie ist in Dünnschichttechnik gefertigt, wobei durch den schichtweisen Aufbau die Leiter innerhalb der Platine und insbesondere auch die Kontaktflächen an der Oberseite der Platine, die in Figur 5 sichtbar ist, gebildet sind. Im vorliegenden Ausführungsbeispiel weist die Platine eine Dicke von 60 µm auf, ihre Breite ist geringfügig schmaler als die eines darauf angeordneten bildaufnehmenden Sensors 9, dessen rückseitige Kontakte mit der Oberseite der Platine 8 elektrisch leitend verbunden sind. Weiterhin sind an der Oberseite in den Figuren 5 und 6 mit Abstand rechts und links zum Sensor 9 Kondensatoren 10 auf der Oberseite der Platine 8 angeordnet und elektrisch leitend mit dieser verbunden. Schließlich sind an der Oberseite der Platine 8 elektrische Kontakte 11 vorgesehen, an denen einadrige elektrisch isolierte Kabel 12 ebenfalls durch Löten angeschlossen werden, über welche die Baugruppe 1 elektrisch leitend mit einer proximalseitig angeordneten (nicht dargestellten) Steuer- und Auswerteinheit verbunden ist.

Die Kondensatoren 10, die mit Abstand und parallel zum Sensor 9 auf der Oberseite der Platine 8 angeordnet sind, erstrecken sich also quer zur Längsrichtung 13 der Platine 8.

Diese anhand der Figuren 5 und 6 dargestellte und fertig bestückte Platine 8 wird im Bereich des Sensors 9 rückseitig mit einem Verstärkungskörper 14 versehen, der in seiner Kontur geringfügig kleiner als der Sensor 9 ist und gleichzeitig einen Kern bildet, um welchen die Platine 8 wie in Figur 7 dargestellt abgebogen wird. Die Platine wird dabei um eine parallel zur Querachse 15 angeordnete Biegelinie zunächst um etwa 90° zu beiden Seiten des Verstärkungskörpers 14 abgebogen, folgt in diesem Bereich dem Verstärkungskörper 14 über seine Höhe, um dann nochmals zu beiden Seiten jeweils um 90° abgebogen zu werden und bis zur rückseitigen Mitte längs des Verstärkungskörpers 14 geführt zu werden. Dort ist die Platine 8 wiederum zu beiden Seiten um 90° abgebogen, derart, dass die freien Platinenenden parallel zueinander etwa aneinander anliegend sich von der Mitte des Verstärkungskörpers 14 proximalwärts erstrecken.

Die Biegestellen, die durch randseitige Ausnehmungen 16 im Querschnitt geschwächt sind, sind so angeordnet, dass der Verstärkungskörper 14 von dem mittleren Teil der Platine 8 an vier Seiten nahezu vollständig umgeben wird. Dabei ist der Abstand der Kondensatoren 10 zum Sensor 9 so gewählt, dass diese wie in den Figuren 4 und 7 erkennbar, in gebogenem Zustand der Platine 8 mit Abstand hinter dem Bereich angeordnet sind, in welchem die Platine 8 den Verstärkungskörper 14 umgibt. Am Ende der Platine 8 sind die Kontakte 11 angeordnet, die, da die Platine 8 mit ihren Enden aneinander liegend nach dem Faltvorgang ausläuft, dann auf zwei gegenüber liegend abgewandten Seiten der Baugruppe 1 angeordnet sind.

Dabei können die Kabel 7 wie vorbeschrieben und anhand von Figur 3 dargestellt, an den Kontakten 11 angelötet sein. Es kann jedoch auch vorgesehen sein, die Platine so auszugestalten, wie dies anhand von Figur 2 dargestellt ist, d. h., dass alle Kontakte in Längsrichtung der Baugruppe 1 gesehen hintereinander liegen. Die Platine 8 kann auch so ausgebildet sein, dass sie die Leitungsverbindung bis zum proximalen Ende des Optikkanals 4 bildet.

Die anhand von Figur 7 dargestellte Einheit bestehend aus der um den Verstärkungskörper 14 gebogenen Platine 8, bestückt mit dem bildaufnehmenden Sensor 9 sowie den Kondensatoren 10 und den an den Kontakten 11 angeschlossenen Kabeln 12, ist in einem Gehäuse 17 eingegossen, welches den Sensor 9 seitlich umfasst, in diesem Bereich rechteckig ausgebildet ist, verjüngend nach proximalwärts zuläuft, um dort in einen zylindrischen hülsenförmigen Teil 18 überzugehen. Dieses Gehäuse 17 nimmt also die in Figur 4 dargestellte Baueinheit vollständig auf, mit der es durch eine Vergussmasse fest und unlösbar verbunden ist. Schließlich wird das Objektiv 6 mit der bildaufnehmenden Seite des Sensors 9 verbunden, sodass die Baugruppe 1 fertiggestellt ist, welche nicht nur extrem kleine radiale Abmessungen aufweist, sondern auch axiale, wenn man einmal von den im Querschnitt deutlich dünneren Kabeln 12 absieht. Die so gebildete Baugruppe 1 ist sowohl für flexible als auch für starre Endoskope einsetzbar und in ihren radialen Abmessungen nur unwesentlich größer als der bildgebende Sensor im Bereich seiner aktiven Oberfläche.

Die vorbeschriebene Baugruppe 1 ist mit einem Sensor 9 und zwei Kondensatoren 10 sowie einer Vielzahl von Kontakten 11 bestückt. Es versteht sich, dass statt der Kondensatoren 10 ggf. auch andere elektrische oder elektronische Bauteile in diesem Bereich proximalseitig des Verstärkungskörpers 14 angeordnet sein können, auch können Anzahl und Anordnung der Kontakte 11 auf der Platine geeignet variiert werden.

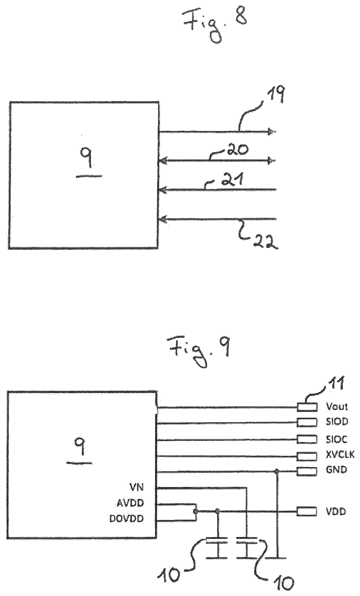

Anhand von Figur 8 ist ein Blockschaltbild zum Anschluss des Sensors 9 dargestellt. Wie daraus ersichtlich ist, weist dieser zum einen einen Videoausgang 19 auf, welcher das eigentliche Bildsignal beinhaltet, darüber hinaus einen Kommunikationsport 20 sowie einen Eingang 21 für die Taktung. Schließlich ist eine Spannungsversorgung 22 vorhanden.

In Stromlaufbahn gemäß Figur 9 sind die Anschlüsse des Sensors 9 im Einzelnen aufgeführt, hierin bedeuten:
- Vout: - Videoausgangssignal
- SIOC SIOD: 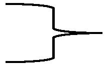 Kommunikationskanal
- XVCLK: - Taktung
- GND: - Spannungsversorgung
- VDD: - Spannungsversorgung
- VN: - Spannungsversorgung
- AVDD: - Spannungsversorgung
- DOVDD: - Spannungsversorgung

In Stromlaufbahn gemäß Figur 9 sind die beiden Kondensatoren 10 deutlich sichtbar, die hier eine Kapazität von jeweils 100 nF aufweisen.

### Bezugszeichenliste

- 1: - Baugruppe
- 2: - distaler Endabschnitt
- 3: - Arbeitskanal
- 4: - Optikkanal
- 5: - Fenster
- 6: - Objektiv
- 7: - Kabel
- 8: - Platine
- 8': - Platine
- 9: - Sensor
- 10: - Kondensator
- 11: - Kontakte
- 12: - einadrige Kabel
- 13: - Längsrichtung
- 14: - Verstärkungskörper
- 15: - Querachse
- 16: - Ausnehmungen
- 17: - Gehäuse
- 18: - hülsenförmiger Teil von 17
- 19: - Videoausgang
- 20: - Kommunikationsport
- 21: - Taktung
- 22: - Spannungsversorgung

## Patentansprüche

1. Baugruppe für ein Videoendoskop mit einem bildaufnehmenden Sensor (9), der auf einer flexiblen Platine (8) elektrisch leitend angeordnet ist, die neben dem Sensor (9) mindestens an einer Seite abgebogen und proximalseitig des Sensors (9) mit mindestens einem elektrischen Kabel (7) leitend verbunden ist, **dadurch gekennzeichnet, dass** die Platine (8) neben dem Sensor (9) mindestens ein weiteres elektronisches Bauteil (10) trägt, welches auf dem abgebogenen Teil der Platine (8) proximalseitig des Sensors (9) angeordnet ist, dass die Platine (8) rückseitig des Sensors (9) mit einem Verstärkungskörper (14) versehen ist, der einen Kern bildet, um welchen die Platine (8) zunächst um etwa 90° zu beiden Seiten des Verstärkungskörpers (14) abgebogen ist, in diesem Bereich dem Verstärkungskörper (14) über seine Höhe folgt, dann nochmals zu beiden Seiten jeweils um 90° abgebogen und bis zur rückseitigen Mitte längs des Verstärkungskörpers (14) geführt und dort wiederum zu beiden Seiten um 90° derart abgebogen ist, dass die freien Platinenenden parallel zueinander anliegend sich von der Mitte des Verstärkungskörpers (14) proximalwärts erstrecken und das mindestens eine weitere elektronische Bauteil (10) tragen.

2. Baugruppe nach Anspruch 1, **dadurch gekennzeichnet, dass** beide Endabschnitte ein weiteres elektronisches Bauteil (10) tragen.

3. Baugruppe nach Anspruch 2, **dadurch gekennzeichnet, dass** beide Endabschnitte jeweils einen Kondensator (10) tragen.

4. Baugruppe nach einem der vorhergehenden Ansprüche, **dadurch gekennzeichnet, dass** die Endabschnitte der Platine (8) an ihren Endabschnitten Kontakte (11) aufweisen, an denen die Kabel (12) angeschlossen sind.

5. Baugruppe nach einem der vorhergehenden Ansprüche, **dadurch gekennzeichnet, dass** die Platine (8) eine vorzugsweise einlagige Flex-Platine mit einer Dicke zwischen 40 µm und 100 µm ist.

6. Baugruppe nach einem der vorhergehenden Ansprüche, **dadurch gekennzeichnet, dass** die Platine (8) in einem Biegeradius abgebogen ist, der dem 0,5 bis 2-fachen, vorzugsweise dem 0,8 bis 1,2-fachen der Platinendicke entspricht.

7. Baugruppe nach einem der vorhergehenden Ansprüche, **dadurch gekennzeichnet, dass** die Platine (8) im Bereich einer Biegung mit mindestens einer Ausnehmung (24) zur Reduzierung der Biegespannung versehen ist.

8. Baugruppe nach einem der vorhergehenden Ansprüche, **dadurch gekennzeichnet, dass** die Platine (8) über einadrige Kabel (12) angeschlossen ist.

9. Baugruppe nach einem der vorhergehenden Ansprüche, **dadurch gekennzeichnet, dass** die platinenseitigen Kabelenden und die Endabschnitte der Platine (8) durch einen abschirmenden Schlauch umgeben sind.

10. Baugruppe nach einem der vorhergehenden Ansprüche, **dadurch gekennzeichnet, dass** der Sensor (9) umfang- und platinenseitig die Platine (8) und die platinenseitigen Kabelenden mit dem abschirmenden Schlauch, vorzugsweise in einem umgebenden Gehäuse (17) in Kunststoff vergossen sind.

11. Baueinheit nach einem der vorhergehenden Ansprüche, **dadurch gekennzeichnet, dass** sie aus der um einen Verstärkungskörper (14) gebogenen Platine (8), bestückt mit dem bildaufnehmenden Sensor (9) sowie den Kondensatoren (10) und den an den Kontakten (11) angeschlossenen Kabeln (12) besteht und in einem Gehäuse (17) eingegossen ist, welches den Sensor (9) seitlich umfasst, in diesem Bereich rechteckig ausgebildet ist, verjüngend nach proximalwärts zuläuft, um dort in einen zylindrischen hülsenförmigen Teil (18) überzugehen.

12. Endoskop mit einem starren oder flexiblen Schaft mit einer Baugruppe (1) nach einem der vorhergehenden Ansprüche, **dadurch gekennzeichnet, dass** die Baugruppe (1) in einem distalen Abschnitt (2) des Schaftes angeordnet und die Kabel (12) durch den Schaft zum proximalen Ende geführt sind.

## Claims

1. A subassembly for a video endoscope with a picture-recording sensor (9) which is electrically conductively arranged on a flexible circuit board (8) which next to the sensor (9) is bent at least at one side and proximally of the sensor (9) is conductively connected to at least one electrical cable (7), **characterised in that** the circuit board (8) apart from the sensor (9) carries at least one further electronic component (10) which is arranged on the bent part of the circuit board (8) proximally of the sensor (9), that the circuit board (8) at the rear side of the sensor (9) is provided with a reinforcement body (14) which forms a core, around which the circuit board (8) is firstly bent by about 90° at both sides of the reinforcement body (14), follows the reinforcement body (14) over its height in this region, then is once again bent at both sides each by 90° and is led along the reinforcement body (14) up to the rear-side middle and there is again bent by 90° at both sides in a manner such that the free circuit board ends, bearing parallel to one another, extend proximally from the middle of the reinforcement body (14) and carry the at least one further electronic component (10).

2. A subassembly according claim 1, **characterised in that** both end sections carry a further electronic component (10).

3. A subassembly according to claim 2, **characterised in that** both end sections each carry a capacitor (10).

4. A subassembly according to one of the preceding claims, **characterised in that** the end sections of the circuit board (8) at their end sections comprise contacts (11), to which the cables (12) are connected.

5. A subassembly according to one of the preceding claims, **characterised in that** the circuit board (8) is a preferably single-layer flex circuit board with a thickness between 40 µm and 100 µm.

6. A subassembly according to one of the preceding claims, **characterised in that** the circuit board (8) is bent with a bending radius which corresponds to 0.5 to 2-fold, preferably 0.8 to 1.2-fold the circuit board thickness.

7. A subassembly according to one of the preceding claims, **characterised in that** the circuit board (8) in the region of a bending is provided with at least one recess (24) for reducing the bending stress.

8. A subassembly according to one of the preceding claims, **characterised in that** the circuit board (8) is connected via a single-conductor cable (12).

9. A subassembly according to one of the preceding claims, **characterised in that** the circuit-board-side cable ends and the end sections of the circuit board (8) are surrounded by a shielding sheath.

10. A subassembly according to one of the preceding claims, **characterised in that** the sensor (9) at the peripheral side and circuit board side, the circuit board (8) and the cable ends which are at the circuit board side and are with the shielding sheath are preferably moulded in plastic in a surrounding housing (17).

11. A subassembly according to one of the preceding claims, **characterised in that** it consists of the circuit board (8) which is bent around a reinforcement body (14), equipped with the picture-recording sensor (9) as well as with the capacitors (10) and of the cables (12) which are connected to the contacts (11) and is moulded in a housing (17) which laterally encompasses the sensor (9), is designed rectangularly in these regions, tapers proximally so as to a merge into a cylindrical sleeve-like part (18).

12. An endoscope with a rigid or flexible shank with a subassembly (1) according to one of the preceding claims, **characterised in that** the subassembly (1) is arranged in a distal section (2) of the shank, and the cables (12) are led through the shank to the proximal end.

## Revendications

1. Module pour un vidéo-endoscope avec un capteur d'image (9) qui est disposé de manière électriquement conductrice sur une platine flexible (8) qui est repliée à côté du capteur (9) sur au moins un côté et qui est reliée de manière conductrice du côté proximal du capteur (9) à au moins un câble électrique (7), **caractérisé en ce que** la platine (8) porte outre le capteur (9) au moins un autre composant électronique (10) qui est disposé du côté proximal du capteur (9) sur la partie repliée de la platine (8), **en ce que** la platine (8) est pourvue du côté arrière du capteur (9) d'un corps de renforcement (14) qui forme un noyau autour duquel la platine (8) est repliée d'abord d'environ 90° de chaque côté du corps de renforcement (14) puis suit dans cette zone le corps de renforcement (14) sur sa hauteur, puis est encore repliée de 90° de chaque côté et est emmenée jusqu'au milieu arrière du corps de renforcement (14) et y est à nouveau repliée de 90° de chaque côté de façon telle que les extrémités libres de la platine s'étendent parallèlement l'une à l'autre et de manière adjacente, du milieu du corps de renforcement (14) en direction proximale et portent ledit au moins un composant électronique supplémentaire (10).

2. Module selon la revendication 1, **caractérisé en ce que** les deux zones d'extrémité portent un composant électronique supplémentaire (10).

3. Module selon la revendication 2, **caractérisé en ce que** les deux zones d'extrémité portent chacune un condensateur (10).

4. Module selon l'une des revendications précédentes, **caractérisé en ce que** les zones d'extrémité de la platine (8) comprennent à leurs zones d'extrémité des contacts (11) auxquels les câbles (12) sont raccordés.

5. Module selon l'une des revendications précédentes, **caractérisé en ce que** la platine (8) est une platine flexible à, de préférence, une couche avec une épaisseur entre 40 µm et 100 µm.

6. Module selon l'une des revendications précédentes, **caractérisé en ce que** la platine (8) est repliée avec un rayon de courbure qui correspond à 0,5 à 2 fois, de préférence à 0,8 à 1,2 fois, l'épaisseur de la platine.

7. Module selon l'une des revendications précédentes, **caractérisé en ce que** la platine (8) est pourvue, dans la zone d'une courbure, d'au moins un évidement (24) pour réduire la contrainte de flexion.

8. Module selon l'une des revendications précédentes, **caractérisé en ce que** la platine (8) est raccordée par des câbles (12) à un seul fil.

9. Module selon l'une des revendications précédentes, **caractérisé en ce que** les terminaisons de câbles du côté platine et les zones d'extrémité de la platine (8) sont entourées d'un tube de blindage.

10. Module selon l'une des revendications précédentes, **caractérisé en ce que** le capteur (9) sur le pourtour et du côté de la platine, la platine (8) et les terminaisons de câbles du côté de la platine avec le tuyau de blindage, sont coulés dans de la matière synthétique, de préférence dans un boîtier (17) les entourant.

11. Unité modulaire selon l'une des revendications précédentes, **caractérisée en ce qu'**elle est constituée d'une platine (8) repliée autour d'un corps de renforcement (14) et pourvue du capteur d'image (9) ainsi que des condensateurs (10) et des câbles (12) raccordés aux contacts (11), et est coulée dans un boîtier (17) qui comprend le capteur (9) sur le côté, et qui présente dans cette zone une forme rectangulaire, se rétrécissant en direction proximale, afin de continuer dans une partie (18) en forme de manchon cylindrique.

12. Endoscope ayant une tige rigide ou flexible avec une unité modulaire (1) selon l'une des revendications précédentes, **caractérisé en ce que** l'unité modulaire (1) est disposée dans une partie distale (2) de la tige et que les câbles (12) sont passés par la tige vers l'extrémité proximale.
